# FASCICULE DE BREVET EUROPEEN

(11) **EP 4 347 542 B1**
(45) Date de publication et mention de la délivrance du brevet: **16.07.2025**
(21) Numéro de dépôt: 22730245.2
(22) Date de dépôt: 24.05.2022
(51) Int. Cl.: C07C 2/32, C07C 11/107, B01J 8/22, B01J 10/00

(54) **PROCEDE D'OLIGOMERISATION DANS UN REACTEUR A ZONES DE DIAMETRES VARIABLES COMPRENANT UNE ETAPE DE RECYCLAGE D'UN SOLVANT PREALABLEMENT REFROIDI**
VERFAHREN ZUR OLIGOMERISIERUNG IN EINEM REAKTOR MIT ZONEN MIT VARIABLEM DURCHMESSER MIT EINEM SCHRITT ZUR RÜCKFÜHRUNG EINES VORGEKÜHLTEN LÖSUNGSMITTELS
METHOD FOR OLIGOMERISATION IN A REACTOR COMPRISING VARIABLE-DIAMETER ZONES, INCLUDING A STEP OF RECYCLING A PRE-COOLED SOLVENT

(30) Priorité: 28.05.2021 FR 2105617
(43) Date de publication de la demande: 10.04.2024
(73) Titulaire: IFP Energies nouvelles, 92500 Rueil-Malmaison (FR)
(72) Inventeur: COTTE, Olivier, 92852 Rueil-Malmaison Cedex (FR); BREUIL, Pierre-Alain, 92852 Rueil-Malmaison Cedex (FR)
(74) Mandataire: IFP Energies nouvelles
(86) Numéro de dépôt international: PCT/EP2022/064008
(87) Numéro de publication internationale: WO 2022/248449

(56) Documents cités:
- EP-A1- 2 703 373
- CN-U- 210 855 896
- DE-C1- 4 338 414
- FR-A1- 3 068 620

## Description

### Domaine technique

La présente invention concerne un procédé d'oligomérisation d'une charge oléfinique mis en oeuvre dans un réacteur à zones de diamètre variable dans lequel une fraction solvant issue d'une étape aval de séparation est refroidie et recyclée. En particulier, la présente invention concerne un procédé d'oligomérisation d'une charge oléfinique gazeuse, de préférence d'éthylène gazeux, en alpha-oléfines linéaires telles que le but-1-ène, le hex-1-ène, ou l'oct-1-ène ou un mélange d'alpha-oléfines linéaires.

### Art antérieur

L'invention concerne le domaine de l'oligomérisation visant à produire des alpha-oléfines utilisées comme co-monomère des procédés de production de polyéthylène. La réaction d'oligomérisation est couramment réalisée dans un procédé de catalyse homogène en phase liquide dans un réacteur bi-phasique gaz/liquide, en général avec une mise en oeuvre dans une colonne à bulles.

Le brevet DE4338414C1 décrit un procédé d'oligomérisation d'une charge oléfinique, comprenant :a) Une étape d'oligomérisation de la charge oléfinique, dans une section réactionnelle ainsi qu' une étape de refroidissement d'un effluent de réaction et une étape de séparation d'un effluent de réaction issu de l'étape a)d'oligomérisation dans une section de séparation de façon à obtenir une fraction solvant (9),et une étape d'introduction dans la section réactionnelle de l'étape a)d'oligomérisation de la fraction solvant.

La réaction d'oligomérisation est fortement exothermique, il est courant de réguler la température de réaction par la mise en oeuvre d'un refroidissement externe. Un réacteur peut être couplé à une ou plusieurs boucle(s) de recirculation afin de soutirer une fraction liquide, de la refroidir par un ou des échangeurs, et de la réintroduire dans le réacteur. Ladite boucle de recirculation permet d'obtenir une bonne homogénéité des concentrations et de contrôler la température dans l'ensemble du volume réactionnel. Le brevet EP2703373 propose un procédé de trimérisation de l'éthylène en hexène-1 permettant de diminuer le coût des installations en limitant la consommation énergétique liée à la boucle de recirculation. Pour cela, une fraction de fond composée principalement de solvant issue de la section de séparation est utilisée dans l'échangeur thermique de la boucle de recirculation et également pour le rebouillage du fond d'une colonne de la section de séparation.

Un inconvénient rencontré lors de la mise en œuvre de réacteur bi-phasique gaz/liquide dans des procédés d'oligomérisation par exemple d'éthylène est la gestion du ciel gazeux, correspondant à la partie supérieure du réacteur à l'état gazeux. Ledit ciel gazeux comprend les composés gazeux peu solubles dans la phase liquide, des composés partiellement solubles dans le liquide mais inertes, ainsi que de l'éthylène gazeux non dissous dans ledit liquide. Le passage de l'éthylène gazeux de la partie inférieure liquide de l'enceinte réactionnelle vers le ciel gazeux est un phénomène appelé perçage. Or le ciel gazeux est purgé afin d'éliminer lesdits composés gazeux. Lorsque la quantité d'éthylène gazeux présente dans le ciel gazeux est importante la purge du ciel gazeux entraine une perte en éthylène non négligeable ce qui nuit à la productivité et au coût du procédé d'oligomérisation. De plus, un phénomène de perçage important signifie que beaucoup d'éthylène gazeux n'a pas été dissous dans la phase liquide et donc n'a pas pu réagir ce qui nuit à la productivité et à la sélectivité du procédé d'oligomérisation.

Afin d'améliorer l'efficacité du procédé d'oligomérisation en termes de productivité et de coût, il est donc indispensable de limiter le phénomène de perçage de l'éthylène afin d'améliorer sa conversion dans ledit procédé tout en conservant une bonne sélectivité en alpha oléfines linéaires souhaitées.

Dans le domaine de l'invention, l'homme du métier cherche constamment à améliorer les procédés d'oligomérisation notamment en maîtrisant le dimensionnement des équipements ayant un impact sur les performances et le coût du procédé. Il cherche aussi à réduire le coût des installations mises en œuvre pour réaliser l'oligomérisation.

La demanderesse a découvert un procédé d'oligomérisation d'une charge oléfinique mis en œuvre dans un réacteur à zones de diamètre variable et dans lequel une fraction solvant issue d'une étape aval de séparation est refroidie et recyclée de manière à contrôler en partie l'exothermie générée par la réaction d'oligomérisation dans le réacteur. La présente invention a pour but d'améliorer le procédé d'oligomérisation d'une charge oléfinique, en particulier de l'éthylène, dans un réacteur gaz/liquide. Elle cherche notamment à améliorer la productivité/la rentabilité du procédé, notamment afin d'éviter le phénomène de perçage et/ou afin de limiter les coûts d'investissement et/ou d'exploitation du procédé. La mise en œuvre du recyclage d'une fraction refroidie de solvant issue d'une section de séparation dans le procédé selon l'invention permet de limiter la taille du ou des échangeurs de chaleur utilisés dans au moins une boucle de recirculation. La mise en œuvre d'un réacteur à zones de diamètre variable selon l'invention permet d'améliorer la dissolution de la charge oléfinique gazeuse et donc de limiter le phénomène de perçage.

### Description sommaire de l'invention

La présente invention porte sur un procédé d'oligomérisation d'une charge oléfinique, comprenant :
a) Une étape d'oligomérisation de la charge oléfinique, mise en oeuvre à une température comprise entre 30 et 200°C et une pression comprise entre 0,1 et 10 MPa, en présence d'un système catalytique d'oligomérisation homogène et d'un solvant, dans une section réactionnelle comprenant :
   un réacteur d'oligomérisation à zones de diamètre variable et comprenant une phase liquide, et
   au moins une boucle de recirculation permettant le refroidissement d'au moins une partie d'une fraction de la phase liquide à une température T_{boucle},
b) Une étape de séparation d'un effluent de réaction issu de l'étape a) d'oligomérisation dans une section de séparation de façon à obtenir une fraction solvant,
c) Une étape de refroidissement de la fraction solvant issue de l'étape b) à une température inférieure à la température T_{boucle} à laquelle la fraction de la phase liquide est refroidie dans la ou les boucle(s) de recirculation,
d) Une étape d'introduction dans la section réactionnelle de l'étape a) d'oligomérisation de la fraction solvant refroidie issue de l'étape c).

De préférence, ledit réacteur à zones de diamètre variable comprend n zones consécutives, n étant un entier positif compris entre 2 et 10, avec :
- pour chacune des n zones ayant un diamètre Dn décroissant dans le sens de la zone de fond vers la zone du sommet dudit réacteur,
- un rapport (Dn/Dn-1) du diamètre de la zone supérieure, noté Dn, sur le diamètre de la zone inférieure adjacente, noté Dn-1, est inférieur ou égale à 0,9
- pour une zone donnée, un rapport entre le volume de ladite zone, noté Vn, sur le volume total du réacteur, noté Vtot, compris entre 0,2 et 0,8.

De préférence, les n zones consécutives du réacteur à zones de diamètre variable sont disposées en série selon l'axe vertical du réacteur de manière à définir des zones dans l'enceinte réactionnelle ayant des diamètres décroissants du fond vers le sommet.

De préférence, la fraction solvant issue de l'étape b) est refroidie dans l'étape c) à une température comprise entre 0 et 150°C.

De préférence, la fraction solvant issue de l'étape b) est refroidie à l'étape c) à une température inférieure d'au moins 40°C par rapport à la température T_{boucle} de la fraction de la phase liquide refroidie dans la ou les boucle(s) de recirculation.

De préférence, le refroidissement de la fraction solvant à l'étape c) est réalisé par un ou plusieurs échangeur(s) thermique(s), de préférence choisi(s) parmi un ou plusieurs échangeurs de chaleur du type fluide process/fluide process, de type aéroréfrigérant, de type échangeur à eau de refroidissement.

De préférence, la section de séparation comprend au moins deux colonnes de distillation, de préférence au moins trois colonnes de distillation, de préférence au moins quatre colonnes de distillation.

De préférence, l'étape d) d'introduction de la fraction solvant refroidie est réalisée dans le réacteur et/ou dans une ou plusieurs des boucle(s) de recirculation.

De préférence, l'étape d) d'introduction d'au moins une partie de la fraction solvant refroidie est réalisée dans une boucle de recirculation en amont ou en aval d'un échangeur thermique de la ou des boucle(s) de recirculation, de préférence en aval dudit échangeur thermique.

De préférence, la fraction solvant refroidie présente un débit en un pourcentage massique par rapport au débit de la fraction de la phase liquide circulant dans la ou les boucle(s) de recirculation compris entre 0,05 et 15,0%, de préférence entre 0,1 et 10,0%.

De préférence, la charge oléfinique comprend des oléfines ayant entre 2 et 6 atomes de carbone, de préférence entre 2 et 4 atomes de carbone.

De préférence, l'étape a) d'oligomérisation comprend au moins une des sous-étapes suivantes
- Etape a1) d'introduction du système catalytique,
- Etape a2) de mise en contact avec de la charge oléfinique,
- sous-étape étape a3) de soutirage d'une fraction de la phase liquide du réacteur d'oligomérisation,
- sous-étape a4) de refroidissement d'au moins une partie de la fraction de la phase liquide soutirée à l'étape a3) dans au moins une boucle de recirculation, à une température T_{boucle},
- Etape a5) d'introduction dans le réacteur de la fraction liquide refroidie.

De préférence, la sous étape a4) de refroidissement est mise en oeuvre par la circulation d'au moins une partie de la fraction de la phase liquide soutirée à l'étape a3), au travers d'un ou plusieurs échangeurs thermiques situés dans la ou les boucles de recirculation.

De préférence, le ou les échangeurs thermiques mis en oeuvre à la sous-étape a4) diminue la température de la fraction de la phase liquide soutirée à la sous-étape a3) de 1,0 à 30,0°C, de préférence entre 2,0 et 25,0°C.

De préférence, l'effluent de réaction est obtenu par division en deux flux de la fraction liquide soutirée à l'étape a3).

### Définition

Dans le cadre de la présente invention, le terme « section de séparation » désigne le ou les dispositifs de séparation, notamment par distillation, disposés en aval de la section réactionnelle, avec un seul dispositif ou une pluralité de dispositifs disposés en série et/ou en parallèle, dispositifs qui peuvent être identiques ou différents par leur dimensionnement ou leur conception/fonctionnement.

Dans le cadre de la présente invention, on comprend les termes « amont » et « aval » en fonction du sens général d'écoulement du fluide réactionnel dans l'unité de production.

Dans le cadre de la présente invention, les expressions échangeur de chaleur et échangeur thermique sont utilisées de façon équivalente.

On entend par catalyseur ou système catalytique homogène, le fait que le catalyseur ou le système catalytique soit dans la même phase que les réactifs et les produits de la réaction d'oligomérisation.

On entend par perçage le passage de la charge oléfinique gazeuse, de préférence de l'éthylène gazeux, de la phase liquide vers la phase gazeuse contenue dans un réacteur gaz/liquide.

On désigne par taux de solvant, le ratio massique du débit total de solvant injecté sur la somme du débit total de la charge oléfinique gazeuse, de préférence d'éthylène gazeux, injectée et du débit de solvant introduit dans le réacteur.

On entend par réacteur biphasique gaz/liquide un réacteur comprenant une phase liquide et une phase gazeuse, la phase liquide comprenant la charge oléfinique de préférence sous forme gazeuse, en particulier de l'éthylène gazeux, les produits de la réaction tels que l'alpha oléfine linéaire souhaitée (*i.e.* butène-1, hexène-1, octène-1 ou le mélange d'alpha-oléfines linéaires), de préférence sous forme liquide, le système catalytique, de préférence sous forme liquide, et un solvant, et une phase gazeuse se situant dans la partie se situant au sommet du réacteur.

Dans le cadre de la présente invention, le terme « section réactionnelle » désigne un dispositif comprenant, de préférence consistant en, un réacteur d'oligomérisation à zones de diamètre variables et une ou des boucle(s) de recirculation.

On entend par fond ou partie inférieure de l'enceinte réactionnelle ou du réacteur, le quart inférieur de l'enceinte réactionnelle ou du réacteur.

On entend par sommet de l'enceinte réactionnelle ou du réacteur le quart supérieur de l'enceinte réactionnelle ou du réacteur.

On entend par zone de fond, la première zone selon l'invention se situant dans la partie inférieure de l'enceinte réactionnelle ou du réacteur au niveau du fond de ladite enceinte ou du réacteur.

On entend par zone du sommet, la dernière zone selon l'invention se situant dans la partie supérieure de l'enceinte réactionnelle ou du réacteur au niveau du sommet de ladite enceinte ou du réacteur.

On entend par taux de saturation, le pourcentage de charge oléfinique, de préférence d'éthylène, dissoute dans la phase liquide par rapport à la quantité maximale de charge oléfinique gazeuse, de préférence d'éthylène, qui pourrait être dissoute dans ladite phase liquide, défini par l'équilibre thermodynamique entre la pression partielle de charge oléfinique gazeuse, de préférence d'éthylène gazeux, et ladite phase liquide. Le taux de saturation peut être mesuré par chromatographie en phase gazeuse.

On entend par partie supérieure du réacteur, le quart supérieur dudit réacteur contenant la phase liquide.

On entend par volume de liquide réactionnel, la quantité volumique de phase liquide contenue dans le réacteur et/ou la ou les boucles de recirculation et dans laquelle se produit la réaction d'oligomérisation.

On désigne par volume d'une ou des boucles de recirculation, la taille de la ou lesdites boucles correspondant au volume de liquide réactionnel pouvant être contenu par la ou lesdites boucles.

### Description détaillée

### Procédé

La présente invention concerne un procédé d'oligomérisation d'une charge oléfinique mis en œuvre dans un réacteur à zones de diamètre variable, procédé dans lequel une fraction solvant issue d'une étape aval de séparation est refroidie et recyclée à la section réactionnelle de manière à contrôler en partie l'exothermie de la réaction d'oligomérisation.

En particulier, la présente invention concerne un un procédé d'oligomérisation d'une charge oléfinique, comprenant :
a) Une étape d'oligomérisation de la charge oléfinique, mise en œuvre à une température comprise entre 30 et 200°C et une pression comprise entre 0,1 et 10 MPa, en présence d'un système catalytique d'oligomérisation homogène et d'un solvant, dans une section réactionnelle comprenant :
   un réacteur d'oligomérisation à zones de diamètre variable et comprenant une phase liquide, et
   au moins une boucle de recirculation permettant le refroidissement d'au moins une partie d'une fraction de la phase liquide à une température T_{boucle},
b) Une étape de séparation d'un effluent de réaction issu de l'étape a) d'oligomérisation dans une section de séparation de façon à obtenir une fraction solvant,
c) Une étape de refroidissement de la fraction solvant issue de l'étape b) à une température inférieure à la température T_{boucle} à laquelle la fraction de la phase liquide est refroidie dans la ou les boucle(s) de recirculation,
d) Une étape d'introduction dans la section réactionnelle de l'étape a) d'oligomérisation de la fraction solvant refroidie issue de l'étape c).

L'invention permet de réduire l'écart des températures entre la fraction de la phase liquide soutirée et le fluide utilisée pour l'échange de chaleur au niveau de la ou des boucle(s) de recirculation, tout en minimisant la température du solvant introduit dans le réacteur à zones de diamètre variable par la(ou les) boucle(s) de recirculation et tout en maximisant le volume de liquide réactionnel dans le réacteur ce qui permet d'améliorer la saturation en charge oléfinique gazeuse dudit liquide. Le contrôle de la température par l'introduction dans la section réactionnelle de la fraction solvant refroidie issue de la séparation aval permet de réduire la quantité de calories à échanger au niveau des boucles de recirculation et permet donc de réduire la taille des échangeurs. Le gain sur la surface d'échange des échangeurs thermique de la(ou des) boucle(s) de recirculation peut être avantageusement de l'ordre de 1 à 50%, de préférence entre 2% et 40% et préférentiellement entre 3 et 30% sur la surface d'échange.

Par ailleurs, la réaction d'oligomérisation a lieu à la fois dans le liquide réactionnel contenu dans le réacteur et dans la ou les boucle(s) de recirculation. La réduction de la taille des échangeurs de chaleur signifie que le volume d'au moins une boucle de recirculation diminue. Dans le procédé selon l'invention, le volume de liquide réactionnel dans le réacteur est augmenté par rapport à un procédé ne mettant pas en œuvre l'invention, à volume de liquide réactionnel total (réacteur + boucle de recirculation) identique, ce qui permet d'améliorer la saturation du milieu réactionnel liquide en charge oléfinique et donc les performances du procédé.

Dans le procédé selon l'invention, la proportion de volume de liquide réactionnel dans une boucle de recirculation par rapport au volume total de liquide réactionnel diminue par rapport à un procédé ne mettant pas en œuvre l'invention, à quantité de liquide réactionnel dans le réacteur identique. Cette diminution et en particulier combinée à l'emploi du réacteur à zones de diamètre variable permet de réduire le temps de séjour et donc d'améliorer les performances du système catalytique en termes d'activité et de sélectivité, tout en améliorant la saturation du liquide en charge oléfinique.

Ainsi la présente invention permet de moduler facilement la productivité et la rentabilité du procédé d'oligomérisation en fonction des performances du système catalytique mis en oeuvre.

### Système catalytique d'oligomérisation homogène

Tous les systèmes catalytiques connus de l'Homme du métier et aptes à être mis en oeuvre dans les procédés de dimérisation, de trimérisation, de tétramérisation et plus généralement dans les procédés d'oligomérisation selon l'invention, font partie du domaine de l'invention. Lesdits systèmes catalytiques ainsi que leur mise en oeuvre sont notamment décrits dans les demandes FR2984311, FR2552079, FR3019064, FR3023183, FR3042989 ou encore dans la demande FR3045414.

De préférence, les systèmes catalytiques comprennent, de préférence sont constitués de :
- un précurseur métallique de préférence à base de nickel, de titane ou de chrome,
- optionnellement un agent activateur,
- optionnellement un additif, et
- optionnellement un solvant.

### Le précurseur métallique

Le précurseur métallique utilisé dans le système catalytique est choisi parmi les composés à base de nickel, de titane ou de chrome.

Dans un mode de réalisation, le précurseur métallique est à base de nickel et préférentiellement comprend du nickel de degré d'oxydation (+II). De préférence, le précurseur de nickel est choisi parmi les carboxylates de nickel(II) tel que par exemple le 2-éthylhexanoate de nickel, les phénates de nickel(ll), les naphténates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de π-allylnickel(II), le bromure de π-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydratée ou non, pris seuls ou en mélange.

Dans un second mode de réalisation, le précurseur métallique est à base de titane et préférentiellement comprend un composé aryloxy ou alcoxy du titane.

Le composé alcoxy du titane répond avantageusement à la formule générale [Ti(OR)₄] dans laquelle R est un radical alkyle linéaire ou ramifié. Parmi les radicaux alcoxy préférés, on peut citer à titre d'exemple non limitatifs : le tétraéthoxy, le tétraisopropoxy, le tétra-n-butoxy et le tétra-2-éthyl-hexyloxy.

Le composé aryloxy du titane répond avantageusement à la formule générale [Ti(OR')₄] dans laquelle R' est un radical aryle substitué ou non par des groupements alkyle ou aryle. Le radical R' peut comporter des substituants à base d'hétéroatome. Les radicaux aryloxy préférés sont choisis parmi le phénoxy, le 2-méthylphénoxy, le 2,6-diméthylphénoxy, le 2,4,6-triméthylphénoxy, le 4-méthylphénoxy, le 2-phénylphénoxy, le 2,6-diphénylphénoxy, le 2,4,6-triphénylphénoxy, le 4-phénylphénoxy, le 2-tert-butyl-6-phénylphénoxy, le 2,4-ditertbutyl-6-phénylphénoxy, le 2,6-diisopropylphénoxy, le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy, le radical biphénoxy, le binaphtoxy, le 1,8-naphtalène-dioxy.

Selon un troisième mode de réalisation, le précurseur métallique est à base de chrome et préférentiellement comprend un sel de chrome (II), un sel de chrome (III), ou un sel de degré d'oxydation différent pouvant comporter un ou plusieurs anions identiques ou différents, tels que par exemple des halogénures, des carboxylates, des acétylacétonates, des anions alcoxy ou aryloxy. De préférence, le précurseur à base de chrome est choisi parmi CrCl₃, CrCl₃(tétrahydrofurane)₃, Cr(acétylacétonate)₃, Cr(naphténate)₃, Cr(2-éthylhexanoate)₃, Cr(acétate)₃.

La concentration en nickel, en titane ou en chrome, est comprise entre 0,001 et 300,0 ppm en masse de métal atomique par rapport à la masse réactionnelle, de préférence entre 0,002 et 100,0 ppm, préférentiellement entre 0,003 et 50,0 ppm, plus préférentiellement entre 0,05 et 20,0 ppm et encore plus préférentiellement entre 0,1 et 10,0 ppm en masse de métal atomique par rapport à la masse liquide réactionnel, c'est-à-dire à la masse de phase liquide contenue dans le réacteur et/ou la ou les boucles de recirculation.

### L'agent activateur

Optionnellement, quel que soit le précurseur métallique, le système catalytique comprend un ou plusieurs agents activateurs choisis parmi les composés à base d'aluminium tels que le dichlorure de méthylaluminium (MeAlCl₂), le dichloroéthylaluminium (EtAlCl₂), le sesquichlorure d'éthylaluminium (Et₃Al₂Cl₃), le chlorodiéthylaluminium (Et₂AlCl), le chlorodiisobutylaluminium (i-Bu₂AlCl), le triéthylaluminium (AlEt₃), le tripropylaluminium (Al(n-Pr)₃), le triisobutylaluminium (Al(i-Bu)₃), le diéthyl-éthoxyaluminium (Et₂AlOEt), le méthylaluminoxane (MAO), l'éthylaluminoxane et les méthylaluminoxanes modifiés (MMAO).

### L'additif

Optionnellement, le système catalytique comprend un ou plusieurs additifs.

L'additif est choisi parmi les composés phosphorés monodentés, des composés phosphorés bidentés, des composés phosphorés tridentés, des composés oléfiniques, des composés aromatiques, des composés azotés, des bipyridines, des diimines, des éthers monodentés, des éthers bidentés, des thioéthers monodentés, des thioéthers bidentés, des carbènes monodentés ou bidentés, des ligands mixtes tels que des phosphinopyridines, des iminopyridines, des bis(imino)pyridines

Lorsque le précurseur métallique du système catalytique est à base de nickel, l'additif est choisi parmi,
- les composés de type azoté, tels que la triméthylamine, la triéthylamine, le pyrrole, le 2,5-diméthylpyrrole, la pyridine, la 2-méthylpyridine, la 3-méthylpyridine, la 4-méthylpyridine, la 2-méthoxypyridine, la 3-méthoxypyridine, la 4-méthoxypyridine, la 2-fluoropyridine, la 3-fluoropyridine, la 3-triflurométhylpyridine, la 2-phénylpyridine, la 3-phénylpyridine, la 2-benzylpyridine, la 3,5-diméthylpyridine, la 2,6-diterbutylpyridine et la 2,6-diphénylpyridine, la quinoline, la 1,10-phénanthroline, N-méthylpyrrole, N-butylpyrrole N-méthylimidazole, le N-butylimidazole, la 2,2'-bipyridine, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, ou
- les composés de type phosphine choisi indépendamment parmi la tributylphosphine, la triisopropylphosphine, la tricyclopentylphosphine, la tricyclohexylphosphine, la triphénylphosphine, la tris(o-tolyl)phosphine, le bis(diphénylphosphino)éthane, l'oxyde de trioctylphosphine, l'oxyde de triphénylphosphine, la triphénylphosphite, ou
- les composés répondant à la formule générale (I) ou un des tautomères dudit composé : 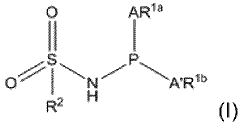 dans laquelle
- A et A', identiques ou différents, sont indépendamment un oxygène ou une liaison simple entre l'atome de phosphore et un atome de carbone,
- les groupements R^{1a} et R^{1b} sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant ou non des hétéroéléments; les groupements phényle, o-tolyle, m-tolyle, p-tolyle, mésityle, 3,5-diméthylphényle, 4-n-butylephényle, 2-méthylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-di(trifluorométhyl)phényle, benzyle, naphthyle, bisnaphthyle, pyridyle, bisphényle, furanyle, thiophényle,
- le groupement R² est choisi indépendamment parmi les groupements méthyle, trifluorométhyle, éthyle, n-propyle, i-propyle, n-butyle, i-butyle, t-butyle, pentyle, cyclohexyle, adamantyle, substitués ou non, contenant des hétéroéléments ou non ; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-bis(trifluorométhyl)phényle, benzyle, naphthyle, bisnaphthyle, pyridyle, bisphényle, furanyle, thiophényle.

Lorsque le précurseur métallique du système catalytique est à base de titane, l'additif est choisi parmi l'éther diéthylique, le diisopropyléther, le dibutyléther, le diphényléther, le 2-méthoxy-2-méthylpropane, 2-methoxy-2-méthylbutane, le diméthoxy-2,2 propane, le di(2-éthylhexyloxy)-2,2 propane, le 2,5-dihydrofurane, le tétrahydrofurane, le 2-méthoxytétrahydrofurane, le 2-méthyltétrahydrofurane, le 3-méthyltétrahydrofurane, le 2,3-dihydropyrane, le tétrahydropyrane, le 1,3-dioxolane, le 1,3-dioxane, le 1,4-dioxane, le diméthoxyéthane, di(2-méthoxyéthyl)éther, le benzofurane, le glyme et le diglyme pris seuls ou en mélange.

Lorsque le précurseur métallique du système catalytique est à base de chrome, l'additif est choisi parmi,
- les composés de type azoté, tels que la triméthylamine, la triéthylamine, le pyrrole, le 2,5-diméthylpyrrole, la pyridine, la 2-méthylpyridine, la 3-méthylpyridine, la 4-méthylpyridine, la 2-méthoxypyridine, la 3-méthoxypyridine, la 4-méthoxypyridine, la 2-fluoropyridine, la 3-fluoropyridine, la 3-triflurométhylpyridine, la 2-phénylpyridine, la 3-phénylpyridine, la 2-benzylpyridine, la 3,5-diméthylpyridine, la 2,6-diterbutylpyridine et la 2,6-diphénylpyridine, la quinoline, la 1,10-phénanthroline, N-méthylpyrrole, N-butylpyrrole N-méthylimidazole, le N-butylimidazole, la 2,2'-bipyridine, la N,N'-diméthyl-éthane-1,2-diimine, la N,N'-di-t-butyl-éthane-1,2-diimine, la N, N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, ou
- les composés aryloxy de formule générale [M(R³O)₂₋ₙXₙ]_{y} dans laquelle
   * M est choisi parmi le magnésium, le calcium, le strontium et le baryum, de préférence le magnésium,
   * R³ est un radical aryl contenant de 6 à 30 atomes de carbone, X est un halogène ou un radical alkyl contenant de 1 à 20 atomes de carbone,
   * n est un nombre entier qui peut prendre les valeurs de 0 ou 1, et
   * y est un nombre entier compris entre 1 et 10, de préférence y est égal à 1, 2, 3 ou 4.

De préférence, le radical aryloxy R³O est choisi parmi le 4-phénylphénoxy, le 2-phénylphénoxy, le 2,6-diphénylphénoxy, le 2,4,6-triphénylphénoxy, le 2,3,5,6-tétraphénylphénoxy, le 2-tert-butyl-6-phénylphénoxy, le 2,4-ditertbutyl-6-phénylphénoxy, le 2,6-diisopropylphénoxy, le 2,6-diméthylphénoxy, le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy. Les deux radicaux aryloxy peuvent être portés par une même molécule, comme par exemple le radical biphénoxy, le binaphtoxy ou le 1,8-naphtalène-dioxy, De préférence, le radical aryloxy R³O est le 2,6-diphénylphénoxy, le 2-tert-butyl-6-phénylphénoxy ou le 2,4-ditert-butyl-6-phénylphénoxy.

### Etape a) d'oligomérisation

Le procédé selon l'invention comprend donc une étape a) d'oligomérisation de la charge oléfinique mise en oeuvre à une température comprise entre 30 et 200°C et une pression comprise entre 0,1 et 10 MPa, en présence d'un système catalytique d'oligomérisation homogène et d'un solvant. Ladite étape a) d'oligomérisation est réalisée dans une section réactionnelle comprenant un réacteur d'oligomérisation à zones de diamètre variable et au moins une boucle de recirculation permettant le contrôle de la température dans ledit réacteur par le refroidissement d'une fraction de phase liquide. Le refroidissement de la fraction de liquide consiste à refroidir ladite fraction à une température inférieure à la température d'oligomérisation (c'est-à-dire à celle de la phase liquide dans le réacteur) de manière à contrôler l'exothermie de la réaction.

De préférence, le réacteur à zones de diamètre variable mis en œuvre dans le procédé selon l'invention comprend n zones consécutives, n étant un entier positif compris entre 2 et 10, avec :
* pour chacune des n zones un diamètre Dn décroissant dans le sens de la zone de fond vers la zone du sommet dudit réacteur,
* un rapport (Dn/Dn-1) du diamètre de la zone supérieure, noté Dn, sur le diamètre de la zone inférieure adjacente, noté Dn-1, est inférieur ou égale à 0,9
* pour une zone donnée un rapport entre le volume de ladite zone, noté Vn, sur le volume total du réacteur, noté Vtot, compris entre 0,2 et 0,8.

La mise en œuvre d'un réacteur à zones de diamètre variable selon la présente invention permet d'augmenter la hauteur totale du réacteur et donc la hauteur de la phase liquide sans modifier le volume du réacteur ni de la phase liquide mis en œuvre dans une réaction d'oligomérisation, ce qui a pour effet d'améliorer la dissolution de la charge oléfinique gazeuse, en particulier de l'éthylène gazeux, et donc de limiter le phénomène de perçage pour un volume de phase liquide donné, et donc d'améliorer la productivité du procédé. Ainsi, le réacteur à zones de diamètre variable permet pour un volume de phase liquide donné, d'augmenter la hauteur de la phase liquide par rapport à un réacteur de diamètre constant.

Avantageusement la mise en œuvre du réacteur à zones de diamètre variable selon l'invention dans un procédé d'oligomérisation, de préférence par catalyse homogène, permet d'avoir un taux de saturation en charge oléfinique, en particulier en éthylène, dissoute dans la phase liquide supérieur à 70,0 %, de préférence entre 70,0 et 100 %, de préférence entre 80,0 et 100 %, de manière préférée compris entre 80,0 et 99,0 %, de préférence entre 85,0 et 99,0 % et de manière encore plus préférée entre 90,0 et 98,0 %.

Le taux de saturation en charge oléfinique, en particulier en éthylène, dissoute peut être mesuré par toute méthode connue de l'Homme du métier et par exemple par l'analyse chromatographique en phase gaz (couramment appelée GC ou CPG) d'une fraction de la phase liquide soutirée du réacteur.

Le procédé mettant en œuvre le réacteur à zones de diamètre variable selon l'invention permet l'obtention d'oléfines linéaires et particulièrement d'alpha-oléfines linéaires par la mise en contact d'oléfine(s) et d'un système catalytique, éventuellement en présence d'un additif et/ou d'un solvant, et par la mise en œuvre dudit réacteur gaz/liquide à zones de diamètre variable.

Les n zones consécutives du réacteur mis en œuvre dans le procédé selon l'invention sont disposées en série selon l'axe vertical du réacteur de manière à définir des zones réactionnelles ayant des diamètres décroissants du fond vers le sommet du réacteur et ainsi d'augmenter la hauteur de la phase liquide pouvant être contenue dans le réacteur selon l'invention par rapport à la hauteur d'un réacteur de diamètre constant, et donc d'augmenter le temps durant lequel la charge oléfinique est présente dans la phase liquide de manière à favoriser sa dissolution.

Avantageusement, pour un volume de réacteur donné et donc un volume de liquide donné, les n zones consécutives de diamètre décroissant dans ledit réacteur permettent d'augmenter la hauteur du liquide pouvant être contenue dans ledit réacteur et ainsi le temps de séjour de la charge oléfinique gazeuse introduite dans ladite phase liquide. Ainsi, la présente invention permet d'augmenter la quantité de charge oléfinique, de préférence d'éthylène, dissoute dans la phase liquide et donc de limiter le phénomène de perçage.

De préférence, le réacteur comprend un nombre n de zones compris entre 2 et 10, de préférence entre 2 et 8, de préférence entre 2 et 6, de manière préférée entre 2 et 5 et de manière très préférée n est égal à 2, 3, 4 ou 5.

Le rapport (Dn/Dn-1) du diamètre d'une zone supérieure n, noté Dn, sur le diamètre de la zone inférieure adjacente n-1, noté Dn-1, est inférieur ou égal à 0,9. De préférence le rapport Dn/Dn-1 est compris entre 0,1 et 0,9, de préférence entre 0,15 et 0,85, de préférence entre 0,2 et 0,8 et de manière préférée entre 0,25 et 0,75 et de manière très préférée entre 0,3 et 0,7.

Les n zones composant le réacteur ont une hauteur totale, notée Htot, dont la somme est égale à la hauteur totale du réacteur.

Avantageusement, le rapport (Hn/Hn-1) de la hauteur d'une zone supérieure n, notée Hn, sur la hauteur de la zone inférieure adjacente n-1, notée Hn-1, est compris entre 0,2 et 3,0, de préférence entre 0,3 et 2,5, de préférence entre 0,4 et 2,0, de préférence entre 0,5 et 1,5 et de manière préférée entre 0,6 et 1,0

De préférence, pour une zone donnée le rapport du volume noté Vn, sur le volume total, noté Vtot, (noté Vn/Vtot) du réacteur correspondant à la somme des n zones est compris entre 0,2 et 0,8. De préférence ledit rapport (Vn/Vtot) est compris entre 0,25 et 0,75, de préférence entre 0,3 et 0,7 et de manière préférée entre 0,35 et 0,65.

De préférence, le réacteur est de forme cylindrique et présente un rapport hauteur totale sur le diamètre de la zone de fond dudit réacteur (noté Htot/D1) compris entre 1 et 17, de préférence entre 1 et 8, et de manière préférée entre 2 et 7.

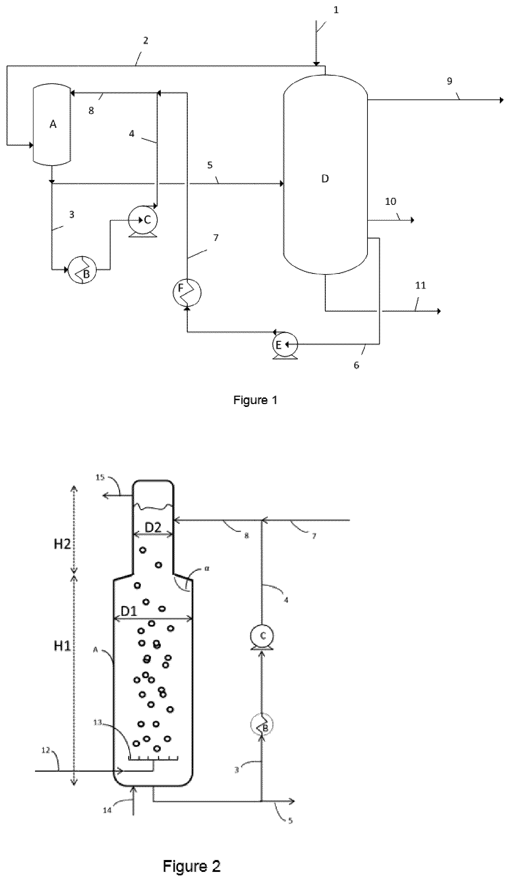

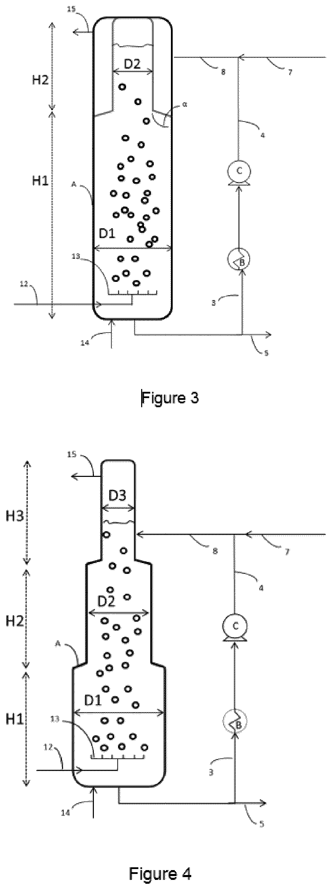

Dans un premier mode de réalisation particulier représenté à la figure 2 ou figure 3, les n zones composant le réacteur selon l'invention sont formées de cylindres de diamètre décroissant. Lesdits cylindres sont reliés entre eux par l'intermédiaire de parois perpendiculaires à l'axe vertical ou présentant un angle α entre 90 et 160° avec l'axe vertical, représenté sur la figure 2, de manière à faciliter et surtout ne pas bloquer l'ascension des bulles d'éthylène gazeux dans la phase liquide. De préférence, ledit angle est compris entre 95 et 145°, et de manière préférée entre 100 et 130°.

Dans un second mode de réalisation particulier représenté à la figure 4, les n zones composant le réacteur selon l'invention sont formées par des internes positionnés à l'intérieur du réacteur de manière à diminuer son diamètre sur une zone donnée. Lesdits internes peuvent être par exemple des parois métalliques pleines.

Avantageusement, quel que soit le mode de réalisation la solidarisation des éléments constituants le réacteur est réalisée par fixation des cylindres et/ou des internes, par exemple par soudage, par collage, par vissage, par boulonnage seul ou en combinaison, ou tout autre moyen analogue. De préférence, la fixation est mise en œuvre par soudage.

De préférence, le réacteur d'oligomérisation est choisi parmi un réacteur biphasique gaz/liquide, de manière préférée un réacteur de type colonne à bulles.

La charge oléfinique comprend de préférence des oléfines ayant entre 2 et 6 atomes de carbone, de préférence entre 2 et 4 atomes de carbone. De préférence, la charge oléfinique est choisie parmi le butène, plus particulièrement l'isobutène ou le butène-1, le propylène, et l'éthylène, seuls ou en mélange.

Dans le reste du présent texte, sauf mention contraire, quand on mentionne spécifiquement l'éthylène, on désigne également les oléfines ayant entre 2 et 6 atomes de carbone, tel que par exemple l'isobutène ou le butène-1, le propylène, et l'éthylène.

De préférence, le procédé d'oligomérisation est un procédé de dimérisation, de trimérisation ou de tétramérisation de la charge oléfinique, de préférence de l'éthylène.

Avantageusement, le procédé d'oligomérisation est mis en œuvre à une pression comprise entre 0,1 et 10,0 MPa, de préférence entre 0,2 et 9,0 MPa et préférentiellement entre 0,3 et 8,0 MPa, à une température comprise entre 30 et 200°C, de préférence entre 35 et 180°C, de préférence entre 45 et 170°C, de préférence entre 60 et 160°C, de préférence entre 70 et 150°C, de préférence entre 80 et 145°C et de manière préférée entre 100 et 140°C.

L'effluent de réaction issu de l'étape a) qui est envoyé vers la section de séparation aval de la section réactionnelle est obtenu par soutirage d'une fraction liquide du réacteur. En particulier, l'effluent de réaction issu de l'étape a) qui est envoyé vers la section de séparation aval de la section réactionnelle correspond à au moins une partie d'une fraction de la phase liquide, soutirée du réacteur d'oligomérisation à zones de diamètre variable. Avantageusement, le débit de l'effluent de réaction est régulé pour maintenir un niveau liquide constant dans le réacteur.

La réaction d'oligomérisation ayant lieu à la fois dans le réacteur et dans la ou les boucle(s) de recirculation, le temps de séjour dans la section réactionnelle s'entend donc sur l'ensemble du volume du réacteur et de la ou des boucle(s) de recirculation composant la section réactionnelle.

Avantageusement, le procédé d'oligomérisation est mis en œuvre avec un taux de solvant compris entre 0 et 90% pds, de préférence entre à 10 et 85% pds, de préférence entre 20 et 80% pds, de préférence entre 30 et 75% pds. Selon un mode de réalisation préféré de l'invention, le procédé d'oligomérisation est mis en œuvre dans un réacteur biphasique gaz/liquide de type colonne à bulles avec une charge oléfinique gazeuse par exemple avec une charge éthylène gazeux. La dissolution des bulles de charge oléfinique, en particulier d'éthylène, dans le milieu réactionnel a lieu dans la colonne à bulles. Plus la hauteur de liquide disponible dans cette colonne est importante plus la dissolution de l'éthylène est proche de la complète saturation. La sélectivité de la réaction vers le produit principal de réaction étant inversement liée à la conversion de la charge oléfinique, de préférence de l'éthylène, dans le liquide, maximiser la quantité de charge oléfinique dissoute permettra à débit constant de charge oléfinique en entrée du réacteur de diminuer la conversion et donc d'augmenter la sélectivité.

Les boucles de recirculation représentant une proportion importante du volume de liquide réactionnel de la section réactionnelle, il est d'intérêt de réduire au maximum ce volume afin :
- Soit d'augmenter le volume de liquide réactionnel dans le réacteur (par exemple dans une colonne à bulles) et en conséquence d'augmenter la hauteur de la phase liquide dans le réacteur, pour maximiser la saturation en éthylène dans le liquide, de manière synergique avec le réacteur à zones de diamètre variable,
- Soit de diminuer le volume de la ou les boucles de recirculation tout en maintenant un volume du réacteur constant et ainsi réduire le temps de séjour, ce qui peut permettre d'améliorer les performances du système catalytique en termes d'activité et de sélectivité.

Le ou les solvants sont avantageusement choisis parmi les éthers, les alcools, les solvants halogénés et les hydrocarbures, saturés ou insaturés, cycliques ou non, aromatiques ou non, comprenant entre 1 et 20 atomes de carbone, de préférence entre 4 et 15 atomes de carbone, préférentiellement entre 4 et 12 atomes de carbone et encore plus préférentiellement entre 4 et 8 atomes de carbone.

De préférence, le solvant est choisi parmi le pentane, l'hexane, le cyclohexane, le méthylcyclohexane, l'heptane, le butane ou l'isobutane, le cycloocta-1,5-diène, le benzène, le toluène, l'ortho-xylène, le mésitylène, l'éthylbenzène, le diéthyléther, le tétrahydrofurane, le 1,4-dioxane, le dichlorométhane, le dichloroéthane, le tétrachloroéthane, l'hexachloroéthane, le chlorobenzène, le dichlorobenzène, le butène, l'hexène et l'octène, purs ou en mélange.

De préférence, le solvant peut être choisi parmi les produits de la réaction d'oligomérisation. De manière préférée, le solvant utilisé est le cyclohexane.

Afin d'évacuer l'énergie de la réaction, une ou plusieurs boucle(s) de recirculation sont utilisées. La boucle de recirculation permet de faire circuler depuis le fond du réacteur, une fraction de phase liquide comprenant les produits de la réaction, le solvant et le système catalytique au travers d'un échangeur avant d'être renvoyée en tête du réacteur.

De préférence, les alpha oléfines linéaires obtenues comprennent de 4 à 20 atomes de carbone, de préférence de 4 à 18 atomes de carbone, de préférence de 4 à 10 atomes de carbone, et de préférence de 4 à 8 atomes de carbone. De manière préférée, les oléfines sont des alpha-oléfines linéaires, choisi parmi le but-1-ène, le hex-1-ène ou l'oct-1-ène.

Avantageusement, la section réactionnelle comprend un ou plusieurs réacteurs de type gaz/liquide ou tout liquide dont au moins un des réacteurs est à zones de diamètre variable, disposé en série et/ou en parallèle, ainsi que leurs équipements associés tels que :
- une ou des boucles de recirculation comprenant un ou plusieurs échangeurs thermiques et associées au/à chacun des réacteurs pour contrôler l'exothermie de la réaction,
- des moyens d'introduction du système catalytique d'oligomérisation dans le ou les sections réactionnelles,
- des moyens externes à la section réactionnelle pour séparer/neutraliser le système catalytique.

Avantageusement, l'étape a) d'oligomérisation comprend au moins une des sous-étapes suivantes
- Etape a1) d'introduction du système catalytique,
- Etape a2) de mise en contact avec de la charge oléfinique,
- Etape a3) de soutirage d'une fraction de phase liquide,
- Etape a4) de refroidissement d'au moins une partie de la fraction de de la fraction liquide,
- Etape a5) d'introduction dans le réacteur de la fraction liquide refroidie.

De préférence, l'étape a) d'oligomérisation comprend les sous-étapes a1), a2), a3), a4) et a5).

Dans un mode de réalisation particulier, l'effluent de réaction issu de l'étape a), et qui est envoyé avantageusement vers la section de séparation aval de la section réactionnelle, est obtenu par division en deux flux de la fraction liquide soutirée à l'étape a3). Le premier flux est envoyé vers l'étape a4) de refroidissement, et le second flux correspond à l'effluent de réaction et est envoyé vers la section aval de séparation. Avantageusement, le débit de l'effluent de réaction est régulé pour maintenir un niveau liquide constant dans le réacteur. De préférence, le débit dudit effluent de réaction est de 5 à 200 fois inférieur au débit liquide envoyé à l'étape a4) de refroidissement (c'est-à-dire au débit de la partie de la fraction de la phase liquide envoyée à l'étape a4). De préférence, le débit dudit effluent de réaction est 5 à 150 fois inférieur, de préférence 10 à 120 fois inférieur et de manière préférée 20 à 100 fois inférieur au débit liquide envoyé à l'étape a4) de refroidissement.

### Etape a1) d'introduction du système catalytique

Avantageusement l'étape d'oligomérisation a) comprend une sous-étape a1) d'introduction d'un système catalytique comprenant un précurseur métallique et avantageusement un agent activateur, éventuellement d'un additif et éventuellement d'un solvant ou d'un mélange de solvants.

De préférence, l'introduction du système catalytique est réalisée en mélange avec la fraction liquide refroidie introduite dans le réacteur à l'étape a5).

De préférence, la pression d'introduction dans le réacteur est comprise entre 0,1 et 10,0 MPa, de préférence entre 0,2 et 9,0 MPa et préférentiellement entre 0,3 et 8,0 MPa.

### Etape a2) de mise en contact avec la charge oléfinique

Avantageusement l'étape d'oligomérisation a) comprend une sous-étape a2) d'introduction de la charge oléfinique avantageusement gazeuse, de préférence de l'éthylène gazeux. De préférence, ladite charge oléfinique est introduite dans la phase liquide au niveau de la partie inférieure du réacteur. La charge oléfinique peut comprendre de la charge fraiche, et de préférence, en mélange avec de la charge oléfinique recyclée d'une étape de séparation aval à l'étape a) d'oligomérisation.

De préférence, lorsque la charge oléfinique introduite est gazeuse, ladite charge est distribuée par dispersion lors de son introduction dans la phase liquide inférieure du réacteur par un moyen apte à réaliser ladite dispersion de manière uniforme sur toute la section du réacteur. De préférence, le moyen de dispersion est choisi parmi un réseau distributeur avec une répartition homogène des points d'injection de la charge oléfinique sur toute la section du réacteur.

De préférence, la charge oléfinique est introduite à un débit compris entre 1 et 250 t/h, de préférence entre 2 et 200 t/h de préférence entre 5 et 100 t/h.

De préférence, le débit de charge oléfinique introduite à l'étape a2) est asservi à la pression dans le réacteur.

Selon un mode particulier de mise en oeuvre de l'invention, un flux d'hydrogène gazeux peut également être introduit dans le réacteur, avec un débit représentant 0,01 à 1,0 % en masse du débit de charge oléfinique entrant. De préférence, le flux d'hydrogène gazeux est introduit par la conduite mise en oeuvre pour l'introduction de la charge oléfinique.

### Etape a3) de soutirage d'une fraction de phase liquide

Avantageusement l'étape d'oligomérisation a) comprend une sous-étape étape a3) de soutirage d'une fraction de phase liquide du réacteur d'oligomérisation de préférence dans la partie inférieure dudit réacteur.

Le soutirage mis en œuvre à l'étape a3) est, de préférence, réalisé sous le niveau de l'injection de la charge oléfinique, et de préférence dans le fond du réacteur. Le soutirage est mis en œuvre par tout moyen apte à réaliser le soutirage et de préférence au moyen d'une conduite combinée à une pompe.

De préférence, le débit de soutirage est compris entre 10 et 10000 t/h, et de préférence entre 100 et 7000 t/h.

### Etape a4) de refroidissement de la fraction liquide

Avantageusement, l'étape d'oligomérisation a) comprend une sous-étape a4) de refroidissement d'au moins une partie de la fraction de la phase liquide soutirée à l'étape a3) à une température T(boucle). De préférence, l'étape de refroidissement est mise en œuvre par la circulation d'au moins une partie de la fraction de la phase liquide soutirée à l'étape a3), au travers un ou plusieurs échangeurs thermiques situés dans la boucle de recirculation.

Avantageusement, le ou les échangeurs de chaleur mis en œuvre à la sous étape a4) permettent de diminuer la température de la fraction de la phase liquide de 1,0 à 30,0°C, de préférence entre 2,0 et 25°C, de préférence entre 3,0 et 20,0°C et de manière préférée entre 5,0 et 15,0°C. Avantageusement le refroidissement de la fraction liquide permet de maintenir la température du milieu réactionnel dans les gammes de température souhaitées pour réaliser la réaction d'oligomérisation au sein du réacteur.

Avantageusement, la mise en œuvre de l'étape de refroidissement du liquide, par l'intermédiaire de la boucle de recirculation permet également d'effectuer l'agitation du milieu réactionnel, et ainsi d'homogénéiser les concentrations des espèces réactives dans tout le volume liquide du réacteur.

### Etape a5) d'introduction de la fraction liquide refroidie

Avantageusement l'étape d'oligomérisation a) comprend une sous-étape a5) d'introduction de la fraction liquide refroidie issue de l'étape a4).

L'introduction de la fraction liquide refroidie issue de l'étape a4) est, de préférence, réalisée dans la phase liquide du réacteur, de préférence dans la partie supérieure dudit réacteur, par tout moyen connu de l'Homme du métier, tel qu'une conduite.

De préférence, le débit d'introduction de la fraction liquide refroidie est compris entre 10 et 10000 t/h, et de préférence entre 100 et 7000 t/h.

Les sous étapes a3) à a5) constituent une boucle de recirculation. Avantageusement, la boucle de recirculation permet également d'assurer, en plus du contrôle de la température dans le réacteur, l'agitation du milieu réactionnel, et ainsi d'homogénéiser les concentrations des espèces réactives dans tout le volume liquide du réacteur.

### Etape b) de séparation aval

Le procédé selon l'invention comprend donc une étape b) de séparation d'un effluent issu de l'étape a) d'oligomérisation dans une section de séparation de façon à obtenir entre autres une fraction solvant.

Ladite fraction solvant est principalement composée de solvant. Avantageusement, la teneur en solvant de ladite fraction solvant est supérieure ou égale à 95% poids, de préférence supérieure à 98% poids et de manière préférée supérieure ou égale à 99% poids.

Typiquement, l'étape de séparation située en aval de la section réactionnelle peut mettre en oeuvre des moyens de séparation, tels que des colonnes de distillation, fonctionnant en série et basées sur les différences de points d'ébullition des composés à séparer. Les composés à séparer comprennent le ou les produits de la réaction d'oligomérisation tels que les alpha oléfines linéaires obtenues, éventuellement la charge oléfinique n'ayant pas réagi, et le ou les solvants.

De préférence, l'étape de séparation inclut également une étape préliminaire de neutralisation du catalyseur. Ainsi, l'étape c) de séparation peut mettre en oeuvre une section de neutralisation du catalyseur située en amont de la section de séparation, ladite section de neutralisation étant avantageusement en aval de la section réactionnelle. Le catalyseur peut ensuite être retiré des produits de la réaction de manière dédiée ou en mélange avec les composés les plus lourds.

De préférence, la section de séparation comprend au moins deux colonnes de distillation, de préférence au moins trois colonnes de distillation, de préférence au moins quatre colonnes de distillation. Lesdites colonnes sont positionnées en parallèle et/ou en série, de préférence en série. Selon une variante préférée, la section de distillation comprend trois colonnes de distillation. La séparation de la fraction solvant peut être réalisée dans l'une quelconque des colonnes de distillation de la section aval de séparation dans la mesure où ladite fraction solvant est principalement composée de solvant afin de pouvoir recycler ladite fraction solvant vers le réacteur d'oligomérisation.

De manière avantageuse, l'étape b) de séparation met en oeuvre une première colonne de distillation à une pression comprise entre 0,1 et 3,0 MPa, de préférence entre 0,5 et 1 MPa, une température de tête de colonne comprise entre 0 et 100°C, de préférence entre 40 et 80°C, et une température de fond de colonne comprise entre 100 et 300°C, de préférence entre 140 et 220°C. Ladite première colonne de distillation permet de séparer la charge oléfinique non convertie, en particulier l'éthylène non converti, dans une fraction de tête du reste des composés dans la fraction de fond.

De manière avantageuse, l'étape b) de séparation met en oeuvre une deuxième colonne de distillation à une pression comprise entre 0 et 2,0 MPa, de préférence entre 0,01 et 1,0 MPa, une température de tête de colonne comprise entre 20 et 150°C, de préférence entre 40 et 130°C, et une température de fond de colonne comprise entre 50 et 300°C, de préférence entre 80 et 250 °C. De manière préférée, ladite deuxième colonne permet de séparer de ladite fraction de fond issue de la première colonne en une fraction de tête comprenant les alpha oléfines linéaires obtenues, en particulier de l'hexène-1, et le solvant, et une fraction de fond comprenant les composés les plus lourds.

De manière avantageuse, l'étape b) de séparation met en oeuvre une troisième colonne de distillation à une pression comprise entre 0 et 1,0 MPa, de préférence entre 0,01 et 0,5 MPa, une température de tête de colonne comprise entre 30 et 130°C, de préférence entre 50 et 90°C, et une température de fond de colonne comprise entre 50 et 200°C, de préférence entre 90 et 180°C. De manière préférée, ladite troisième colonne permet de séparer l'hexène-1 en tête du solvant en fond.

A titre d'exemple non limitatif, dans le cas de la trimérisation de l'éthylène en hexène-1, l'effluent de réaction issu de l'étape a) de trimérisation de l'éthylène comprenant, de l'éthylène, le solvant, le système catalytique de la trimérisation de l'éthylène et les produits formés dont l'hexène-1, peut être séparé dans un étape b) de séparation comprenant au moins les sous-étapes suivantes :
- b1) une première étape de séparation dans une première colonne de distillation, de l'effluent de la réaction de trimérisation de l'éthylène, en une fraction de tête comprenant l'éthylène non converti et une fraction de fond,
- b2) une deuxième étape de séparation dans au moins une autre colonne de distillation d'au moins une partie de la fraction de fond issue de l'étape b1) en une fraction de tête comprenant de l'hexène-1 et le solvant, et une fraction de fond comprenant des hydrocarbures C8+,
- b3) une troisième étape de séparation dans une colonne de distillation finale d'au moins une partie de la fraction comprenant de l'hexène-1 et du solvant issue de l'étape b2) en une fraction de tête comprenant principalement de l'hexène-1 et en une fraction de fond comprenant principalement le solvant, constituant avantageusement au moins en partie la fraction solvant.

Conformément à l'invention, au moins une fraction solvant provenant de la fraction de fond issue de l'étape b3) est refroidie à l'étape c) puis renvoyée dans la section réactionnelle à l'étape d).

### Etape c) de refroidissement

Le procédé selon l'invention comprend une étape c) de refroidissement de la fraction solvant issue de l'étape b) de séparation aval à une température inférieure à la température T_{boucle} à laquelle la fraction de la phase liquide est refroidie dans la ou les boucle(s) de recirculation de la section réactionnelle.

Le refroidissement de la fraction solvant à une température inférieure à la température T_{boucle} de la boucle de recirculation, de préférence de la température de la fraction liquide refroidie obtenue à l'issu de l'étape a4), permet de diminuer l'échange de chaleur, soit la quantité de calories échangée, nécessaire dans la ou les boucles de recirculation pour atteindre la température de la fraction liquide refroidie issue de ladite boucle qui est introduite dans le réacteur, et donc de diminuer la taille du ou des échangeurs.

Le refroidissement de la fraction solvant à l'étape c) peut être réalisé par échange dans un ou plusieurs échangeurs thermiques, soit avec un fluide du procédé, soit avec de l'air, soit avec de l'eau de refroidissement ou tout autre type de fluide froid permettant d'atteindre la température souhaitée ou à l'aide d'une combinaison de ces échangeurs. Avantageusement, l'échangeur est choisi parmi un ou plusieurs échangeurs de chaleur du type fluide process/fluide process (de type TEMA ou autres connus de l'homme du métier), de type aéroréfrigérant, de type échangeur à eau de refroidissement ou tout autre type de fluide froid permettant d'atteindre la température souhaitée.

Avantageusement, la fraction solvant issue de l'étape b) est refroidie à une température comprise entre 0 et 150°C, de préférence entre à 5 et 100°C, de préférence entre 10 et 90°C, de préférence entre 20 et 80°C, de préférence entre 25 et 70°C, et de manière préférée entre 30 et 60°C.

Avantageusement, la fraction solvant issue de l'étape b) est refroidie à l'étape c) à une température inférieure d'au moins 40°C, de préférence d'au moins 50°C, de préférence d'au 60°C, de préférence d'au moins 70°C par rapport à la température T_{boucle} de la fraction liquide refroidie dans la ou les boucle(s) de recirculation de la section réactionnelle.

### Etape d) d'introduction de la fraction issue de c)

Le procédé selon l'invention comprend donc une étape d) d'introduction dans la section réactionnelle de l'étape a) d'oligomérisation de la fraction solvant refroidie à l'étape c) à une température inférieure à la température T(boucle) de la boucle de recirculation.

L'introduction de la fraction solvant refroidie selon l'invention permet de contrôler en partie l'exothermie de la réaction d'oligomérisation et ainsi de limiter la taille du ou des échangeurs de chaleur mis en oeuvre dans au moins une boucle de recirculation

Avantageusement, l'introduction de la fraction solvant refroidie est réalisée dans la section réactionnelle, de préférence dans le réacteur et/ou dans une ou plusieurs des boucle(s) de recirculation. De préférence, l'introduction est réalisée dans une boucle de recirculation, avantageusement en amont ou en aval de l'échangeur thermique de ladite boucle de recirculation, c'est-à-dire en amont ou en en aval de l'étape a4). De manière préférée l'introduction de la fraction solvant refroidie est réalisée dans une boucle de recirculation de la section réactionnelle en aval de l'échangeur thermique de ladite boucle de recirculation.

Avantageusement, l'introduction de la fraction solvant refroidie en aval de l'échangeur thermique de la boucle de recirculation permet de minimiser l'écart de température entre la fraction solvant refroidie et la fraction liquide de la boucle de recirculation. Cela permet également de maximiser l'utilisation de l'échangeur de la ou des boucles de recirculation et donc de minimiser sa taille.

Avantageusement, le mélange de la fraction solvant refroidie à l'étape c) avec la fraction liquide circulant, de préférence en aval de l'échangeur thermique, dans la boucle de recirculation de la section réactionnelle permet de diminuer la température de la fraction liquide de la boucle de recirculation de 0,1 à 20,0°C, de préférence entre 0,2 et 15,0°C et de manière préférée entre 0,5 et 10,0°C.

Avantageusement, la fraction solvant refroidie présente un débit en un pourcentage massique par rapport au débit du liquide circulant dans la boucle de recirculation compris entre 0,05 et 15,0%, de préférence entre 0,1 et 10,0%, de préférence entre 0,5 et 8,0%, de préférence entre 0,8 et 6,0% et de manière préférée entre 1,0 et 5,0%.

Ainsi la mise en oeuvre des étapes c) et d) selon l'invention permet par l'intermédiaire de la fraction solvant refroidie de réduire l'énergie échangée au niveau des boucles de recirculation, et permet donc réduire la taille de l'échangeur. Le gain attendu peut être avantageusement de l'ordre de 1 à 50%, de préférence entre 2% et 30% et préférentiellement entre 3 et 20% sur la surface d'échange.

### Description des figures

La figure 1 représente une illustration schématique d'une installation mettant en oeuvre un mode de réalisation du procédé d'oligomérisation selon l'invention. Ladite installation comprend un réacteur d'oligomérisation biphasique liquide/gaz A dont les zones de diamètre variable ne sont pas représentées, une boucle de recirculation comprenant un échangeur B et une pompe C, une section de séparation D, une pompe E de circulation de la fraction solvant, un échangeur de refroidissement de la fraction solvant (F). Dans cette installation, le flux 2 est un mélange du flux 1 d'éthylène frais et d'éthylène issu de la section de séparation. Le flux 2 est introduit dans le réacteur A. Le flux 3 est la fraction de phase liquide soutirée du réacteur et envoyée dans la boucle de recirculation comprenant un échangeur B et une pompe C pour obtenir une fraction liquide refroidie 4. Le flux 6 est la fraction de solvant séparée dans la section de séparation D qui passe par une pompe E et est refroidie dans un échangeur F en une fraction solvant refroidie 7. Les fractions 7 et 4 sont mélangées en un flux 8 avant d'être introduit dans le réacteur A. Le flux 5 correspondant à une partie de l'effluent soutiré du réacteur A est envoyé vers la section de séparation D. La section de séparation permet d'obtenir le flux 6, un flux 9 correspondant aux produits de réaction légers, un flux 10 correspondant aux produits de réaction lourds et un flux 11 correspondant à une fraction lourde comprenant le catalyseur usé.

La figure 2 illustre un réacteur gaz/liquide A à zones consécutives de diamètre décroissant selon l'invention comprenant une partie inférieure comprenant une phase liquide, une partie supérieure comprenant une phase gazeuse, et un moyen d'introduction d'une charge oléfinique gazeuse 12 par l'intermédiaire d'un distributeur gazeux 13 dans la phase liquide. La partie supérieure comprend un moyen de purge 15. Dans le fond du réacteur A est soutirée une fraction divisée en deux flux, un premier flux principal 3 envoyé vers un échangeur à chaleur B et une pompe C pour obtenir une fraction refroidie 4. Le second flux 5 correspond à l'effluent envoyé vers la section de séparation. Les fractions 7 et 4 sont mélangées en un flux 8 avant d'être introduit dans le réacteur A. Dans le fond du réacteur est introduit le système catalytique 14. La zone 1 située en fond du réacteur à un diamètre supérieur à la zone située au sommet du réacteur. La première zone de fond est caractérisée par son diamètre noté D1 et sa hauteur H1, ces deux paramètres définissant le volume, noté V1, de ladite zone. De manière similaire, la deuxième zone située au sommet est caractérisée par sa hauteur notée H2 et son diamètre noté D2, inférieur à D1, définissant le volume, V2, de la deuxième zone. Dans ce mode de réalisation, les deux zones composant le réacteur A sont formées de cylindres de diamètre décroissant.

La figure 3 illustre un autre mode de réalisation qui diffère de celui de la figure 2 en ce que la deuxième zone située au sommet du réacteur A est délimitée par un interne 11 positionné à l'intérieur du réacteur A.

La figure 4 illustre un autre mode de réalisation qui diffère de celui de la figure 2 en ce que le réacteur A comprend trois zones consécutives de diamètre décroissant.

Les figures 1, 2, 3 et 4 illustrent schématiquement des modes de réalisation particuliers de l'objet de la présente invention sans en limiter la portée.

### EXEMPLES

Les exemples ci-dessous illustrent l'invention sans en limiter la portée.

Les exemples ci-après décrivent un procédé d'oligomérisation de l'éthylène mis en oeuvre en continu dans un réacteur biphasique gaz/liquide de type colonne à bulles à une pression de 6,1 MPa et une température de 135°C. Le système catalytique est introduit dans le réacteur à une concentration de 1 ppm en poids de chrome, comprend le précurseur de chrome Cr(2-éthylhexanoate)₃, du 2-5-dimethylpyrrole à un ratio molaire par rapport au chrome de 3, 11 équivalents molaires de triéthylaluminium et 8 équivalents molaires de chlorure de diéthylaluminium par rapport au chrome en présence de o-xylène comme additif à un ratio molaire de 500 par rapport au chrome et de cyclohexane comme solvant.

La quantité de cyclohexane, utilisé comme solvant 6, introduit dans le réacteur A est dépendante de la quantité d'éthylène entrant dans le même réacteur A (flux 2), la quantité de solvant est ajustée de manière à avoir un taux de solvant de 58% dans le réacteur.

### Exemple 1 (comparatif)

L'exemple 1 illustre un procédé d'oligomérisation selon l'art antérieur dans lequel la fraction solvant (7) est séparée dans une section aval de séparation et recyclée vers la section réactionnelle sans être refroidie par un échangeur de chaleur F et dans lequel le procédé d'oligomérisation met en oeuvre un réacteur gaz-liquide de type colonne à bulle.

Le système catalytique est mis en contact avec de l'éthylène gazeux par introduction dudit éthylène gazeux dans la partie inférieure dudit réacteur. L'effluent réactionnel est ensuite récupéré en fond de réacteur.

La production d'hexène-1 nécessite la conversion de 14000 kg/h d'éthylène. Le débit de solvant dans les conditions d'opération retenues est de 19500 kg/h. La température de la fraction solvant recyclée est de 101°C.

Le temps de séjour dans la section réactionnelle (réacteurs + boucle(s) de recirculation) est de 40 minutes.

La réaction d'oligomérisation étant exothermique, la chaleur de la réaction est évacuée par des échangeurs de chaleur placés sur des boucles de recirculation extérieures au réacteur, de 1650 m² de surface totale. Le volume de liquide réactionnel total de 41,4m³ est réparti entre le volume pris par les échangeurs de chaleurs et leur boucle de recirculation, et le réacteur. Le volume de liquide réactionnel total se décompose de la façon suivante : 30,4m³ pour les boucles d'échange de chaleur, et 11,0m³ pour le réacteur. La hauteur de liquide dans le réacteur est alors de 4,8 mètres (noté m) pour un diamètre de 1,7 m. La température du mélange 8 de la fraction solvant 7 et du fluide de recirculation 4 est alors, en entrée de réacteur, de 120°C. La température du flux 4, correspondant au flux en sortie de l'échangeur B de la boucle de recirculation de la section réactionnelle, est alors de 120,4°C.

La production en hexène-1 est de 9,32 tonnes/heure, la sélectivité en hexène-1 est de 93,2% poids.

### Exemple 2 (selon l'invention)

Le procédé d'oligomérisation selon l'invention est illustré à la Figure 1 et est mis en oeuvre dans les mêmes conditions que dans l'exemple 1. L'exemple 2 comprend une étape de refroidissement de la fraction solvant issue de la section de séparation avant introduction dans la section réactionnelle, grâce à un échangeur F, telle qu'illustrée dans la figure 1 et un réacteur A à zones de diamètres variables. Ladite fraction solvant est refroidie à une température de 40°C.

La surface totale des échangeurs des boucles de recirculation est de 1440m². Ainsi, le procédé selon l'invention permet de diminuer la surface d'échange des échangeurs de chaleurs d'environ 13% (=100x(1650-1440)/1650) par rapport à la surface d'échange de l'exemple 1, ce qui représente un gain sur le coût d'opération de l'unité.

Dans cet exemple, le temps de séjour est conservé identique à celui de l'exemple 1, soit 40 minutes (mn). Le volume de liquide réactionnel total est identique à celui l'exemple 1. L'étape de refroidissement de la fraction solvant permet donc de réduire le besoin d'échange dans les boucles de recirculation qui se traduit par une diminution de la surface des échangeurs.

Du fait de la diminution de la surface requise dans les échangeurs, le volume des boucles de recirculation est réduit de 3% (volume de 29,5m³). Le volume de liquide du réacteur peut alors être augmenté de 8% (volume utile de 11,9m³) ce qui conduit à un gain sur la hauteur de liquide de 8%.

Par ailleurs l'utilisation d'un réacteur à zones diamètres variables permet d'obtenir un gain supplémentaire de 35% de la hauteur liquide (Hauteur totale de liquide de 7,1m). La hauteur de liquide de la zone inférieure du réacteur est de 2,1m pour un diamètre de 1,7m. La hauteur de la partie supérieure du réacteur liquide est alors de 4,9m pour un diamètre de 1,35m.

La cible de température du mélange 8 de la fraction solvant 7 et de la fraction liquide 4 circulant dans les boucles de recirculation a été conservée identique à l'exemple 1, à savoir 120°C. La température de la fraction liquide refroidie 4, correspondant à la sortie de l'échangeur B, est alors de 121,9°C.

De plus, la diminution de 3% du volume des boucles de recirculation et la mise en oeuvre d'un réacteur gaz/liquide à zones de diamètres variables permet également d'augmenter de 9% le volume de liquide du réacteur et de 47% la hauteur liquide, ce qui permet de maximiser la saturation en éthylène dans le liquide contenu dans le réacteur.

La production en héxène-1 est de 9,32 tonnes/heure, la sélectivité en héxène-1 est de 93,2% poids.

### Exemple 3 (selon l'invention)

Le procédé d'oligomérisation selon l'invention est mis en oeuvre dans les mêmes conditions que dans l'exemple 2.

L'étape de refroidissement de la fraction solvant permet de réduire le besoin d'échange dans les boucles de recirculation, qui se traduit par une diminution de la surface des échangeurs. La fraction solvant est refroidie à une température de 40°C. La surface totale des échangeurs de la boucle d'échange est alors limitée à 1440 m². Ainsi, le procédé selon l'invention permet de diminuer la surface d'échange des échangeurs de chaleurs d'environ 13% (=100x(1650-1440)/1650) par rapport à la surface d'échange de l'exemple 1, ce qui représente un gain sur le coût d'opération de l'unité.

Les hauteur liquide dans le réacteur est conservé identique à l'exemple 1. Le réacteur comporte alors deux zones, une zone inférieure de 1,6m de hauteur pour un diamètre de 1,7m et une zone supérieure de 3,1m de hauteur pour un diamètre de 1,35m. Du fait de la diminution de la surface requise dans les échangeurs, le volume des boucles de recirculation est réduit de 3% (volume de 29,5 m³). Le volume de liquide du réacteur à zones de diamètres variables est de 8,4 m³. Le volume de liquide réactionnel total de la section réactionnelle est donc alors de 37,8 m³, soit 8% de gain par rapport à l'exemple 1.

La cible de température du mélange 8 du solvant 7 et du fluide de recirculation 4 a été conservée identique à l'exemple 1, à savoir 120°C. La température du flux 4, correspondant à la sortie de l'échangeur B, est alors de 121,9°C.

Dans cet exemple, le temps de séjour est de 36,6 minutes. Le procédé selon l'invention permet une diminution du temps de séjour ce qui entraine un gain de sélectivité de 0,1%. Ce gain permet pour une production d'hexene-1 constante, une diminution de la consommation d'éthylène de 0,1% et donc un gain sur le coût d'opération de l'unité. La diminution du temps de séjour permet également un gain sur la concentration en chrome nécessaire à la réalisation de ces performances de 8% (correspondant à 4 ppm de chrome), soit un gain sur la consommation catalytique et donc un gain sur le coût d'opération de l'unité.

La production en héxène-1 est de 9,32 tonnes/heure, la sélectivité en héxène-1 est de 93,3% poids.

Le tableau ci-dessous résume les résultats obtenus pour les exemples 1 à 3.

| | | Exemple 1 | Exemple 2 | Exemple 3 |
|---|---|---|---|---|
| Température de la fraction solvant recyclée | °C | 101 | 40 | 40 |
| Température de la fraction liquide refroidie | °C | 120,4 | 121,9 | 121,9 |
| Température fraction solvant + fraction liquide refroidie | °C | 120 | 120 | 120 |
| Surface échange des boucles de recirculation | m² | 1650 | 1440 | 1440 |
| Volume des boucles de recirculation | m³ | 30,4 | 29,5 | 29,5 |
| Volume de liquide du réacteur | m³ | 11,0 | 11,9 | 8,4 |
| Volume section réactionnelle | m³ | 41,3 | 41,3 | 37,8 |
| Diamètre de la zone inférieure du Réacteur | m | 1,7 | 1,7 | 1,7 |
| Hauteur liquide dans la zone inférieure du Réacteur | m | 4,8 | 2,1 | 1,6 |
| Diamètre de la zone supérieure du Réacteur | m | - | 1,35 | 1,35 |
| Hauteur liquide dans la zone supérieure du Réacteur | | - | 4,9 | 3,1 |
| Hauteur total liquide | | 4,8 | 7,1 | 4,8 |
| Temps de séjour | mn | 40 | 40 | 36,6 |
| Sélectivité | % | 93,2 | 93,2 | 93,3 |

## Revendications

1. Procédé d'oligomérisation d'une charge oléfinique, comprenant :
a) Une étape d'oligomérisation de la charge oléfinique, mise en œuvre à une température comprise entre 30 et 200°C et une pression comprise entre 0,1 et 10 MPa, en présence d'un système catalytique d'oligomérisation homogène et d'un solvant, dans une section réactionnelle comprenant :
un réacteur d'oligomérisation à zones de diamètre variable et comprenant une phase liquide, et
au moins une boucle de recirculation permettant le refroidissement d'au moins une partie d'une fraction de la phase liquide à une température T_{boucle},
b) Une étape de séparation d'un effluent de réaction issu de l'étape a) d'oligomérisation dans une section de séparation de façon à obtenir une fraction solvant,
c) Une étape de refroidissement de la fraction solvant issue de l'étape b) à une température inférieure à la température T_{boucle} à laquelle la fraction de la phase liquide est refroidie dans la ou les boucle(s) de recirculation,
d) Une étape d'introduction dans la section réactionnelle de l'étape a) d'oligomérisation de la fraction solvant refroidie issue de l'étape c).

2. Procédé selon la revendication 1 dans lequel le réacteur à zones de diamètre variable comprend n zones consécutives, n étant un entier positif compris entre 2 et 10, avec :
- pour chacune des n zones ayant un diamètre Dn décroissant dans le sens de la zone de fond vers la zone du sommet dudit réacteur,
- un rapport (Dn/Dn-1) du diamètre de la zone supérieure, noté Dn, sur le diamètre de la zone inférieure adjacente, noté Dn-1, est inférieur ou égale à 0,9
- pour une zone donnée, un rapport entre le volume de ladite zone, noté Vn, sur le volume total du réacteur, noté Vtot, compris entre 0,2 et 0,8.

3. Procédé selon la revendication 2 dans lequel les n zones consécutives du réacteur à zones de diamètre variable sont disposées en série selon l'axe vertical du réacteur de manière à définir des zones dans l'enceinte réactionnelle ayant des diamètres décroissants du fond vers le sommet.

4. Procédé selon l'une quelconque des revendications précédentes dans lequel la fraction solvant issue de l'étape b) est refroidie dans l'étape c) à une température comprise entre 0 et 150°C.

5. Procédé selon l'une quelconque des revendications précédentes dans lequel la fraction solvant issue de l'étape b) est refroidie à l'étape c) à une température inférieure d'au moins 40°C par rapport à la température T_{boucle} de la fraction de la phase liquide refroidie dans la ou les boucle(s) de recirculation.

6. Procédé selon l'une quelconque des revendications précédentes dans lequel le refroidissement de la fraction solvant à l'étape c) est réalisé par un ou plusieurs échangeur(s) thermique(s), de préférence choisi(s) parmi un ou plusieurs échangeurs de chaleur du type fluide process/fluide process, de type aéroréfrigérant, de type échangeur à eau de refroidissement.

7. Procédé selon l'une quelconque des revendications précédentes dans lequel la section de séparation comprend au moins deux colonnes de distillation, de préférence au moins trois colonnes de distillation, de préférence au moins quatre colonnes de distillation.

8. Procédé selon l'une quelconque des revendications précédentes dans lequel l'étape d) d'introduction de la fraction solvant refroidie est réalisée dans le réacteur et/ou dans une ou plusieurs des boucle(s) de recirculation.

9. Procédé selon l'une quelconque des revendications 5 à 7 dans lequel l'étape d) d'introduction d'au moins une partie de la fraction solvant refroidie est réalisée dans une boucle de recirculation en amont ou en aval d'un échangeur thermique de la ou des boucle(s) de recirculation, de préférence en aval dudit échangeur thermique.

10. Procédé selon l'une quelconque des revendications précédentes dans lequel la fraction solvant refroidie présente un débit en un pourcentage massique par rapport au débit de la fraction de la phase liquide circulant dans la ou les boucle(s) de recirculation compris entre 0,05 et 15,0%, de préférence entre 0,1 et 10,0%.

11. Procédé selon l'une quelconque des revendications précédentes dans lequel la charge oléfinique comprend des oléfines ayant entre 2 et 6 atomes de carbone, de préférence entre 2 et 4 atomes de carbone.

12. Procédé selon l'une quelconque des revendications précédentes dans lequel l'étape a) d'oligomérisation comprend au moins une des sous-étapes suivantes
- Etape a1) d'introduction du système catalytique,
- Etape a2) de mise en contact avec de la charge oléfinique,
- sous-étape étape a3) de soutirage d'une fraction de la phase liquide du réacteur d'oligomérisation,
- sous-étape a4) de refroidissement d'au moins une partie de la fraction de la phase liquide soutirée à l'étape a3) dans au moins une boucle de recirculation, à une température T_{boucle},
- Etape a5) d'introduction dans le réacteur de la fraction liquide refroidie.

13. Procédé selon la revendication 12 dans lequel la sous étape a4) de refroidissement est mise en œuvre par la circulation d'au moins une partie de la fraction de la phase liquide soutirée à l'étape a3), au travers d'un ou plusieurs échangeurs thermiques situés dans la ou les boucles de recirculation.

14. Procédé selon la revendication 13 dans lequel le ou les échangeurs thermiques mis en œuvre à la sous-étape a4) diminue la température de la fraction de la phase liquide soutirée à la sous-étape a3) de 1,0 à 30,0°C, de préférence entre 2,0 et 25,0°C.

15. Procédé selon l'une quelconque des revendications 12 à 14 dans lequel l'effluent de réaction est obtenu par division en deux flux de la fraction liquide soutirée à l'étape a3).

## Patentansprüche

1. Verfahren zur Oligomerisierung eines olefinischen Einsatzmaterials, umfassend:
a) Einen Schritt zur Oligomerisierung des olefinischen Einsatzmaterials, der bei einer Temperatur zwischen 30 und 200 °C und einem Druck zwischen 0,1 und 10 MPa in Gegenwart eines homogenen katalytischen Oligomerisierungssystems und eines Lösungsmittels in einem Reaktionsabschnitt durchgeführt wird, der umfasst:
einen Oligomerisierungsreaktor mit Zonen mit variablem Durchmesser und umfassend eine flüssige Phase, und
mindestens einen Rückführungskreis, der das Kühlen mindestens eines Teils einer Fraktion der flüssigen Phase auf eine Temperatur T_{boucle} ermöglicht,
b) Einen Schritt des Trennens eines aus dem Schritt a) zur Oligomerisierung hervorgegangenen Reaktionsaustrags in einem Trennabschnitt, so dass eine Lösungsmittelfraktion erhalten wird,
c) Einen Schritt des Kühlens der aus dem Schritt b) hervorgegangenen Lösungsmittelfraktion auf eine Temperatur, die geringer als die Temperatur T_{boucle} ist, auf welche die Fraktion der flüssigen Phase in dem oder den Rückführkreis(en) gekühlt wird,
d) einen Schritt des Einleitens der aus dem Schritt c) hervorgegangenen gekühlten Lösungsmittelfraktion in den Reaktionsabschnitt des Schritts a) zur Oligomerisierung.

2. Verfahren nach Anspruch 1, wobei der Reaktor mit Zonen mit variablem Durchmesser n aufeinander folgende Zonen umfasst, wobei n eine positive ganze Zahl zwischen 2 und 10 ist, mit:
- für jede der n Zonen einen in Richtung von der Sumpfzone zur Kopfzone des Reaktors hin abnehmenden Durchmesser Dn,
- einem Verhältnis (Dn/Dn-1) des Durchmessers der oberen Zone, bezeichnet als Dn, zum Durchmesser der angrenzenden unteren Zone, bezeichnet als Dn-1, kleiner oder gleich 0,9
- für eine gegebene Zone einem Verhältnis zwischen dem Volumen der Zone, bezeichnet als Vn, zum Gesamtvolumen der Zone, bezeichnet als Vtot, zwischen 0,2 und 0,8.

3. Verfahren nach Anspruch 2, wobei die n aufeinander folgenden Zonen des Reaktors mit Zonen mit variablem Durchmesser in Reihe entlang der vertikalen Achse des Reaktors angeordnet sind, so dass Zonen in der Reaktionskammer definiert werden, die vom Sumpf zum Kopf hin abnehmende Durchmesser haben.

4. Verfahren nach einem der vorhergehenden Ansprüche, wobei die aus dem Schritt b) hervorgegangene Lösungsmittelfraktion in dem Schritt c) auf eine Temperatur zwischen 0 und 150 °C gekühlt wird.

5. Verfahren nach einem der vorhergehenden Ansprüche, wobei die aus dem Schritt b) hervorgegangene Lösungsmittelfraktion im Schritt c) auf eine Temperatur gekühlt wird, die um mindestens 40 °C geringer als die Temperatur T_{boucle} der Fraktion der flüssigen Phase ist, die in dem oder den Rückführungskreis(en) gekühlt wird.

6. Verfahren nach einem der vorhergehenden Ansprüche, wobei das Kühlen der Lösungsmittelfraktion im Schritt c) durch einen oder mehrere Wärmetauscher ausgeführt wird, der(die) vorzugsweise aus einem oder mehreren Wärmetauscher(n) vom Typ Prozessfluid/Prozessfluid, vom Rückkühlertyp, vom Kühlwassertauschertyp ausgewählt ist(sind).

7. Verfahren nach einem der vorhergehenden Ansprüche, wobei der Trennabschnitt mindestens zwei Destillationskolonnen, vorzugsweise mindestens drei Destillationskolonnen, vorzugsweise mindestens vier Destillationskolonnen umfasst.

8. Verfahren nach einem der vorhergehenden Ansprüche, wobei der Schritt d) des Einleitens der gekühlten Lösungsmittelfraktion in dem Reaktor und/oder in einem oder mehreren der Rückführungskreis(e) ausgeführt wird.

9. Verfahren nach einem der Ansprüche 5 bis 7, wobei der Schritt d) des Einleitens mindestens eines Teils der gekühlten Lösungsmittelfraktion in einem Rückführungskreis stromauf oder stromab eines Wärmetauschers des oder der Rückführungskreise(s), vorzugsweise stromab des Wärmetauschers ausgeführt wird.

10. Verfahren nach einem der vorhergehenden Ansprüche, wobei die gekühlte Lösungsmittelfraktion einen Volumenstrom in einem Massenprozentsatz bezogen auf den Volumenstrom der Fraktion der flüssigen Phase, die in dem oder den Rückführungskreis(en) zirkuliert, zwischen 0,05 und 15,0 %, vorzugsweise zwischen 0,1 und 10,0 % aufweist.

11. Verfahren nach einem der vorhergehenden Ansprüche, wobei das olefinische Einsatzmaterial Olefine umfasst, die zwischen 2 und 6 Kohlenstoffatome, vorzugsweise zwischen 2 und 4 Kohlenstoffatome aufweisen.

12. Verfahren nach einem der vorhergehenden Ansprüche, wobei der Schritt a) zur Oligomerisierung mindestens einen der folgenden Teilschritte umfasst
- Schritt a1) des Einleitens des katalytischen Systems,
- Schritt a2) des Inkontaktbringens mit olefinischem Einsatzmaterial,
- Teilschritt Schritt a3) des Entnehmens einer Fraktion der flüssigen Phase aus dem Oligomerisierungsreaktor,
- Teilschritt a4) des Kühlens mindestens eines Teils der im Schritt a3) entnommenen Fraktion der flüssigen Phase in mindestens einem Rückführungskreis auf eine Temperatur T_{boucle},
- Schritt a5) des Einleitens der gekühlten flüssigen Fraktion in den Reaktor.

13. Verfahren nach Anspruch 12, wobei der Teilschritt a4) des Kühlens durch die Zirkulation mindestens eines Teils der im Schritt a3) entnommenen Fraktion der flüssigen Phase durch einen oder mehrere in dem oder den Rückführungskreisen gelegene Wärmetauscher hindurch durchgeführt wird.

14. Verfahren nach Anspruch 13, wobei der oder die im Teilschritt a4) eingesetzten Wärmetauscher die Temperatur der im Teilschritt a3) entnommenen Fraktion der flüssigen Phase um 1,0 bis 30,0 °C, vorzugsweise zwischen 2,0 und 25,0 °C verringern.

15. Verfahren nach einem der Ansprüche 12 bis 14, wobei der Reaktionsaustrag durch Teilung der im Schritt a3) entnommenen flüssigen Fraktion in zwei Ströme erhalten wird.

## Claims

1. Process for the oligomerization of an olefinic feedstock, comprising:
a) a step of oligomerization of the olefinic feedstock, carried out at a temperature between 30°C and 200°C and a pressure between 0.1 and 10 MPa, in the presence of a homogeneous catalytic oligomerization system and of a solvent, in a reaction section comprising:
an oligomerization reactor with zones of variable diameter and comprising a liquid phase, and
at least one recirculation loop allowing the cooling of at least a part of a liquid-phase fraction to a temperature Tₗₒₒₚ,
b) a step of separating a reaction effluent resulting from the oligomerization step a), in a separation section so as to obtain a solvent fraction,
c) a step of cooling the solvent fraction resulting from step b) to a temperature below the temperature Tₗₒₒₚ to which the liquid-phase fraction is cooled in the recirculation loop(s),
d) a step of introducing, into the reaction section of the oligomerization step a), the cooled solvent fraction resulting from step c).

2. Process according to Claim 1, in which the reactor with zones of variable diameter comprises n consecutive zones, n being a positive integer between 2 and 10, with:
- for each of the n zones having a diameter Dn which decreases in the direction of the bottom zone to the top zone of said reactor,
- a ratio (Dn/Dn-1) of the diameter of the upper zone, denoted Dn, to the diameter of the adjacent lower zone, denoted Dn-1, of less than or equal to 0.9,
- for a given zone, a ratio of the volume of said zone, denoted Vn, to the total volume of the reactor, denoted Vtot, of between 0.2 and 0.8.

3. Process according to Claim 2, in which the n consecutive zones of the reactor with zones of variable diameter are arranged in series along the vertical axis of the reactor so as to define zones in the reaction enclosure having diameters decreasing from the bottom to the top.

4. Process according to any one of the preceding claims, in which the solvent fraction resulting from step b) is cooled in step c) to a temperature between 0°C and 150°C.

5. Process according to any one of the preceding claims, in which the solvent fraction resulting from step b) is cooled in step c) to a temperature at least 40°C lower relative to the temperature Tₗₒₒₚ of the liquid-phase fraction cooled in the recirculation loop(s).

6. Process according to any one of the preceding claims, in which the cooling of the solvent fraction in step c) is carried out by one or more thermal exchangers, preferably chosen from one or more heat exchangers of process fluid/process fluid type, of air cooler type, of cooling water exchanger type.

7. Process according to any one of the preceding claims, in which the separation section comprises at least two distillation columns, preferably at least three distillation columns, preferably at least four distillation columns.

8. Process according to any one of the preceding claims, in which step d) of introducing the cooled solvent fraction is carried out in the reactor and/or in one or more of the recirculation loops.

9. Process according to any one of Claims 5 to 7, in which step d) of introducing at least a part of the cooled solvent fraction is carried out in a recirculation loop upstream or downstream of a thermal exchanger of the recirculation loop(s), preferably downstream of said thermal exchanger.

10. Process according to any one of the preceding claims, in which the cooled solvent fraction has a flow rate, as a weight percentage relative to the flow rate of the liquid-phase fraction circulating in the recirculation loop(s) of between 0.05% and 15.0%, preferably between 0.1% and 10.0%.

11. Process according to any one of the preceding claims, in which the olefinic feedstock comprises olefins having between 2 and 6 carbon atoms, preferably between 2 and 4 carbon atoms.

12. Process according to any one of the preceding claims, in which the oligomerization step a) comprises at least one of the following substeps:
- step a1) of introducing the catalytic system,
- step a2) of bringing into contact with olefinic feedstock,
- substep step a3) of withdrawing a liquid-phase fraction from the oligomerization reactor,
- substep a4) of cooling at least a part of the liquid-phase fraction withdrawn in step a3) in at least one recirculation loop, to a temperature Tₗₒₒₚ,
- step a5) of introducing the cooled liquid fraction into the reactor.

13. Process according to Claim 12, in which the cooling substep a4) is carried out by circulating at least a part of the liquid-phase fraction withdrawn in step a3) through one or more thermal exchangers located in the recirculation loop(s).

14. Process according to Claim 13, in which the thermal exchanger(s) used in substep a4) decreases the temperature of the liquid-phase fraction withdrawn in substep a3) by 1.0 to 30.0°C, preferably between 2.0 and 25.0°C.

15. Process according to any one of Claims 12 to 14, in which the reaction effluent is obtained by dividing the liquid fraction withdrawn in step a3) into two streams.
